# EUROPEAN PATENT APPLICATION

(11) **EP 1 684 070 A1**
(43) Date of publication of application: **26.07.2006**
(21) Application number: 05001363.0
(22) Date of filing: 24.01.2005
(51) Int. Cl.: G01N 33/50

(54) **Screening method for testing the protective capability of an antigen**

(71) Applicant: GSF-Forschungszentrum für Umwelt und Gesundheit GmbH, 80807 München (DE)
(72) Inventor: Adler, Heiko, 80687 München (DE); Sutter, Gerd, 80803 München (DE); El-Gogo, Susanne, 81377 München (DE); Staib, Caroline, 80803 München (DE)
(74) Representative: Reinhard - Skuhra - Weise & Partner

(57) **Abstract**

The present invention is directed to a screening method for testing the efficacy of a vaccine, i.e. in order to allow the determination of the protective capacity of a specific antigen. The present invention further pertains to an antigen or a vaccine identified by this screening method.

## Description

The present invention is directed to a screening method for testing the efficacy of a vaccine, i.e. in order to allow the determination of the protective capacity of a specific antigen. The present invention further pertains to an antigen or a vaccine identified by this screening method.

Herpesviruses are extremely common and around 100 have been identified in a variety of animal species. All of the herpesviruses are members of one family, the Herpesviridae, and have certain characteristics in common, such as their ability to establish latency during primary infection. This means that following initial infection the virus remains dormant, to be reactivated by certain triggers such as an individual's immune status, stress or sunlight.

The herpesviruses have been divided into the alpha-, beta- and gamma-herpesviruses. Members of the alpha-herpesvirus subfamily are classified on the basis of their relatively short reproductive cycle, a variable host range, efficient destruction of infected cells and their ability to establish latent infections primarily in sensory ganglia.

Each of the beta-herpesviruses has a long reproductive cycle and a restricted host range. Infected cells often become enlarged. Latency can be maintained in the white cells of the blood, kidneys, secretory glands and other tissues.

The experimental host range of the gamma-herpesviruses is restricted to the family or order to which the natural host belongs. This subfamily of viruses is specific for either T or B lymphocytes, and latency is often demonstrated in lymphoid tissue. Examples for human gamma-herpesviruses are Human herpesvirus type 8 (HHV-8), also known as Kaposi's sarcoma-associated herpesvirus (KSHV) and Epstein-Barr virus (EBV).

Research was focused on the use of molecular techniques to study the pathogenesis and immune response to this gamma-subgroup of herpesviruses (gamma-herpesviruses). This group of viruses includes clinically important viruses that establish a life-long latent infection within lymphocytes. As mentioned above, there are two human gamma-herpesviruses, the EBV and KSHV, both of which establish latency in B lymphocytes. EBV is associated with infectious mononucleosis as well as numerous human tumours. KSHV infection is detectable in virtually all Kaposi's sarcomas and B-cell primary effusion lymphomas. Important animal gamma-herpesviruses are the related Alcelaphine herpesvirus-1 and Ovine herpesvirus-2 (OvHV-2) that are associated with a fatal lymphoproliferative disease of domestic cattle and deer called malignant catarrhal fever (MCF).

Infection of mice with the murine gamma-herpesvirus, MHV-68, has been developed as a small animal model system. This system has enabled the study of key aspects of gamma-herpesvirus infection in a natural host, in particular the detailed study of the fundamentals of virus-host interactions. Thus, some of the previous research work is focused on the use of murine gamma-herpesvirus-68 (MHV-68) as a small animal model for gamma-herpesvirus pathogenesis and immunology. Murine gamma-herpesvirus MHV-68 is a natural pathogen of free-living murid rodents such as wood mice. Mice inoculated intranasally with MHV-68 establish a productive infection in the lung in alveolar epithelial cells causing an interstitial and peri-bronchiolar pneumonia. This productive phase of infection lasts for around 10 days, during which period the virus spreads via lymph nodes to the spleen. Here it establishes latency predominantly in B cells. This initial latent infection in the spleen causes an intense lymphoproliferation of B cells and T cells alike leading to splenomegaly. Although the precise mechanisms underlying splenomegaly are still being elucidated, it is clear that infected B cells and CD4⁺ "helper" T cells are critical. Also, it is clear that a number of cytokines are secreted in abundance and this cytokine 'storm' almost certainly contributes towards driving lymphoproliferation.

Latently infected cells appear in the first week post-infection, reach peak levels during the second to third week before declining to a basal level (1 in 106 leukocytes) that is maintained indefinitely in the host. Like most virus infections, T cells are critical for the immune-mediated resolution of infection. An important recent finding was that the spleen is not the only site of latency and that MHV-68 also persists indefinitely in lungs. The MHV-68 genome has 118kb of largely unique DNA flanked by terminal repeats and its complete DNA sequence has been determined recently. Of the 80 or so open-reading frames (ORFs) found, most are homologous to other gamma-herpesvirus or cellular genes. However, there are a number of ORFs, termed M1-14 that are unique, that is they have no obvious sequence relationship to virus or cellular genes recorded in current sequence databases. The above information is based on the work of Prof. Stewart's Research Group at the University of Liverpool (Faculty of Medicine) and other groups in the field.

Efficient vaccines against persistent virus infections causing important human diseases such as AIDS or Hepatitis C are urgently needed. Vaccine development has been hampered also by the lack of suitable small animal models to reliably test the protective capacity of candidate vaccines against chronic viral infections.

Therefore, it is an object underlying the present invention to provide a screening method, which allows the convenient and reliable determination of the protective capability of an antigen or a vaccine containing same. It is a further object of the present invention to provide a screening method of this kind, which allows the determination of the acute as well as the chronic or long term protective capacity of an antigen or a vaccine.

These objects are achieved by the subject-matter of the independent claims. Preferred embodiments are set forth in the dependent claims.

The present invention provides an effective and reliable system for determining the protective capability of antigens or vaccines in an *improved in vivo* model in non-human animals. This system is based on the specific characteristics of gamma-herpesviruses, which are exploited in this system.

The choice of a natural pathogen for mammals, such as MHV-68 for mice, that persists lifelong after infection, appears to be a particularly promising candidate for a more relevant model system. Here, infections with recombinant gamma-herpesviruses such as MHV-68 as novel challenge model to test the efficacy of heterologous vaccines were investigated.

By the screening method provided in the present invention, a system is at hand which allows the reliable determination of the protective capability of a certain antigen regarding its acute as well as chronic protectivity. The model system allows the determination of this effect over the whole lifetime of the test animal, i.e. also years following vaccination. Since the long term protection of a vaccine is a question of utmost importance for manufacturer of vaccines, the physicians in charge as well as the patient, an important step forward towards vaccine safety and effective usage thereof is taken by the present invention.

Thus, according to a first aspect, the present invention provides a screening method for testing the protective capacity of a candidate antigen comprising the steps of:
a) administering a candidate antigen to a non-human mammal;
b) administering recombinant gamma-herpesvirus containing the same antigen to said non-human mammal; and
c) determining the viral load of said recombinant gamma-herpesvirus in said non-human animal and thus the efficacy of the candidate antigen to elucidate an immune response and thereby immune protection of said non-human mammal.

The term "antigen" as used herein has the meaning which is common in the pertinent field of the art. An antigen thus is defined as a substance recognized by the immune system as foreign or toxic which elicits an immune response. Antigens include for example bacteria, viruses, and chemicals. If the antigen is part of the same organism, it is called a self antigen. An antigen is any substance that can be recognized as foreign and that will generate an immune response. For more precise definitions of this term in the context of the invention, see below.

The non-human animal preferably is selected from the group consisting of primates, pigs, rodents, e.g. mice, dogs, ruminants, e.g. sheeps, and horses. However, also other non-human mammals can be included in the screening method of the present invention as long host specific gamma-herpesviruses are existing, which can be used in step b) of the screening method.

Furthermore, according to a preferred embodiment, the gamma-herpesvirus is selected from the group consisting of Herpesvirus saimiri (HVS), Herpesvirus ateles (HVA), EBV and EBV homologous viruses, for example Lymphocryptovirus, HHV-8 and HHV-8 homologous viruses, porcine gamma-herpesvirus, ovine herpesvirus, for example ovine herpesvirus-2 (OvHV-2) and murine MHV-68. Murine MHV-68 is most preferred in the present invention.

It is, however, noted that also other gamma-herpesviruses may be used in the screening method of the present invention, whether already known or to be identified in the future. The only requirement is that those gamma-herpesviruses are able to carry the antigen in question and to effectively infect a host animal.

In an embodiment, the candidate antigen used in step a) as defined above is present in form of a nucleic acid, from which the antigen itself is expressed in the host, in form of recombinant bacteria expressing the antigen, of antigen in protein form and/or in form of antigen carried by a viral vector.

In a preferred embodiment, the viral vector is modified vaccinia virus Ankara (MVA). MVA was developed due to concerns with the safety of standard strains of vaccinia virus (VV) and is a highly attenuated virus strain characterized by its restricted replicative capacity *in vitro* and avirulence *in vivo.* It was obtained by long-term serial passages of the Ankara strain of vaccinia virus (CVA) on chicken embryo fibroblasts (for review see Mayr, A., Hochstein-Mintzel, V. and Stickl, H. (1975) Infection 3, 6-14; Swiss Patent No. 568 392). The MVA virus was deposited in compliance with the requirements of the Budapest Treaty at CNCM (Institut Pasteur, Collectione Nationale de Cultures de Microorganisms, 25, rue de Docteur Roux, 75724 Paris Cedex 15) on Dec. 15, 1987 under Depositary No. I-721.

The antigen used in the screening method of the present invention preferably is a protein or polypeptide derived from a pathogenic agent.

Pathogenic agents are to be understood to be viruses, bacteria and parasites which may cause a disease, as well as tumor cells which multiply unrestrictedly in an organism and may thus lead to pathological growths. Examples of such pathogenic agents are described in Davis, B.D. et al., (Microbiology, 3rd ed., Harper International Edition). Preferred genes of pathogenic agents are those of influenza viruses, of measles and respiratory syncytial viruses, of dengue viruses, of human immunodeficiency viruses, for example HIV I and HIV II, of human hepatitis viruses, e.g. HCV and HBV, of herpes viruses, of papilloma viruses, of the malaria parasite Plasmodium falciparum, and of the tuberculosis-causing Mycobacteria.

Preferred genes encoding tumor associated antigens are those of melanoma-associated differentiation antigens, e.g. tyrosinase, tyrosinase-related proteins 1 and 2, of cancer testes antigens, e.g. MAGE-1,-2,-3, and BAGE, of non-mutated shared antigens overexpressed on tumors, e.g. Her-2/neu, MUC-1, and p53.

In the screening method of the invention, the candidate antigen and the gamma-herpesvirus are administered to the non-human mammal preferably by intravenous, intranasal, intraperitoneal, buccal, subcutaneous, intramuscular, peroral administration or by inhalation.

The following ways of administration are, for example, contemplated in the present invention: (1) oral administration, for example, drenches (aqueous or non-aqueous solutions or suspensions), tablets, boluses, powders, granules, pastes; (2) parenteral administration, for example, by subcutaneous, intramuscular or intravenous injection as, for example, a sterile solution or suspension; (3) aerosol, for example, as an aqueous aerosol, liposomal preparation or solid particles containing the compound.

Steps a) and b) of the present as defined above screening method generally are performed subsequently, wherein the time period between the first and the second administration is about 1-5 weeks, preferably 2-4 weeks. However, the time point, when the present method is performed, i.e. when the protective capability is determined is not restricted. It might be useful, from case to case, to wait for months or even years before the second administration step of administering gamma-herpesvirus to the non-human mammal is performed.

In the screening method of the present invention the acute protective capacity of the candidate antigen is tested within a time period of between 4-12, preferably 5-10, most preferably about 6 days following step b) as defined above. The acute protective capacity preferably is tested by determining the titer of gamma-herpesvirus in lung and/or spleen tissue/cells or in other organs, which contain gamma-herpesvirus infected cells. Further organs, which might be infected by the gamma-herpesvirus can be different due to the different ways of administration and can, for example, include liver, kidneys and/or bone marrow. The lower the titer of gamma-herpesviruses, the better the protective capability of the candidate antigen.

To determine virus titers of lungs and spleens, the organs are, for example, homogenized in glass douncers. After two times freezing and thawing the homogenates, a plaque assay is performed with 10-fold dilutions of the supernatants on BHK-21 cells (see chapter Examples, below).

In a preferred embodiment, the chronic (or long term or persistent) protective capacity of the candidate antigen is tested within a time period of between 10-150, preferably 15-20, most preferably about 17 days following step b). The chronic protective capacity is tested in step c) above by determining the degree of spleen enlargement and/or by determining the viral load in splenocytes and/or in dendritic cells and/or macrophages as measured by *ex vivo* reactivation and/or by PCR.

The activity of the T cells in the above step can be determined by methods, which are per se known in the art. Further information can be found in, for example, *Immunology, 5*^{*th*} *edition, 2001*.

According to one embodiment, this determination in step c) defined above can be performed by means of a ⁵¹Cr-release assay. Specifically, the T-cell function can be determined by a ⁵¹Cr-release assay using a T-cell bioassay, which is based on the killing of a target cell by a cytotoxic T cell. This assay is based on the uptake of radioactively labeled sodium chromate, Na₂⁵¹CrO₄, by living cells, which do not again spontaneously release that sodium chromate. When these labeled cells are killed, the radioactive chromate is released and its presence in the supernatant of mixtures of target cells and cytotoxic T cells can be measured.

PCR-methods to be used in the present invention are described for example in Newton, PCR, BIOS Scientific Publishers Limited, 1994 and all subsequent editions.

In a further preferred embodiment, the screening method of the invention comprises determining the protective capacity ex vivo by:
- providing splenocytes from a non-human mammal which was infected with a candidate antigen;
- contacting said splenocytes with cells infected with recombinant gamma-herpesvirus containing the same antigen; and
- determining the degree of cytotoxic T cell activation and/or helper T cell function and/or antibody production.

The candidate antigen in step a) is preferably contained in a vaccine.

To prepare vaccines, the antigen is converted into a physiologically acceptable form. This can be done based on the many years of experience in the preparation of vaccines used for vaccination, for example, against smallpox (Kaplan, Br. Med. Bull. 25, 131-135 [1969]). For example, the antigen or the vector containing same, for example a MVA vector (about 10⁶-10⁸ particles of the recombinant MVA) are freeze-dried in 100ml of phosphate-buffered saline (PBS) in the presence of 2% peptone and 1% human albumin in an ampoule, preferably a glass ampoule. The lyophilisate can contain extenders (such as mannitol, dextran, sugar, glycine, lactose or polyvinylpyrrolidone) or other aids (such as antioxidants, stabilizers, etc.) suitable for parenteral administration. The glass ampoule is then sealed and can be stored, preferably at temperatures below -20°C, for several months.

For vaccination the lyophilisate can be dissolved in 0.1 to 0.2 ml of aqueous solution, preferably physiological saline, and administered parenterally, for example by intradermal inoculation. The vaccine according to the invention is preferably injected intracutaneously. Slight swelling and redness, sometimes also itching, may be found at the injection site (Stickl et al., supra). The mode of administration, the dose and the number of administrations can be optimized by those skilled in the art in a known manner. It is expedient where appropriate to administer the vaccine several times over a lengthy period in order to obtain a high level immune responses against the foreign antigen.

In a second aspect, the present invention provides an antigen or a vaccine having protective capacity against a disease identified by the screening method of the present invention.

All publications, patent applications, patents, and other references mentioned herein are incorporated by reference in their entirety. In case of conflict, the present specification, including definitions, will control. In addition, the materials, methods, and examples are illustrative only and not intended to be limiting.

The invention is now further illustrated by the accompanying drawings, in which the following is shown:
Fig.1 **Growth curve of MHV-68-WT and MHV-68-OVA**
   NIH3T3 cells were infected at a MOI of 0.1 with MHV-68-WT or MHV-68-OVA. Cells were harvested at different timepoints and titers determined on BHK cells.
   *In vitro* MHV-68-OVA shows no growth deficiencies compared to MHV-68-WT.
**Fig.2 Expression of ovalbumin in infected fibroblasts**
   1: cells only; 2: cells infected with MHV-68-WT; 3: cells infected with MHV-68-OVA;
   4: cells infected with MHV-68-OVA (positive control)
   NIH3T3 cells were infected with MHV-68-WT or MHV-68-OVA. 48h after infection cells were harvested and expression of ovalbumin was detected in western blot.
Fig.3 **Detection of OVA-specific, IFNγ secreting CD8+ T cells by intracellular cytokine staining**
   Splenocytes of MVA-WT or MVA-OVA immunized C57B1/6 mice were incubated with MC3T3 fibroblasts infected with different MOI of MHV-68-OVA or MHV-68-WT. Only MVA-OVA immunized mice incubated with MHV-68-OVA infected cells could elicit 4,8% to 8 % activated CD8+ T cells compared to control groups (siinfekl-peptide = positive control).

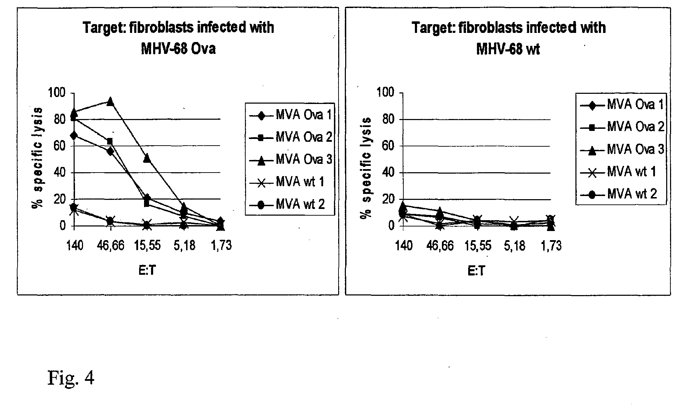

Fig.4 **OVA-specific, MHC class I restricted T cells measured by Cr**^{**51**} **release assay**
   Splenocytes of MVA-WT (data not shown) or MVA-OVA immunized mice were incubated with fibroblasts infected with MHV-68-OVA or MHV-68-WT (MOI of 10). Only T cells of MVA-OVA immunized mice were able to specifically lyse MHV-68-OVA infected fibroblasts.

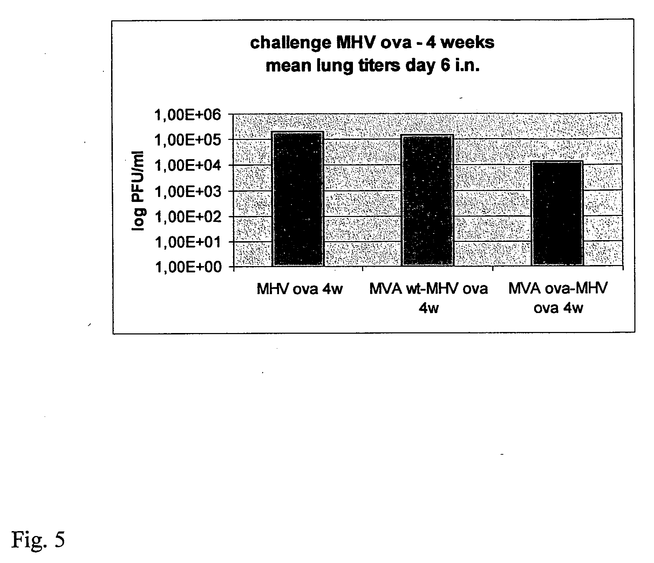

Fig.5 **Mean lung titers of challenge with MHV-68―OVA 4 weeks after prime-boost** immunization with MVA
   C57B1/6 mice (n=3) were immunized two times within 14 days with MVA-WT or MVA-OVA. 4 weeks after second immunization mice were challenged intranasally with MHV-68-OVA and lungs were harvested at day 6 after challenge.
   Mice immunized with MVA-OVA showed specific reduction of lung titer of 1 log compared to control groups that received MVA-WT or MHV-68-OVA.

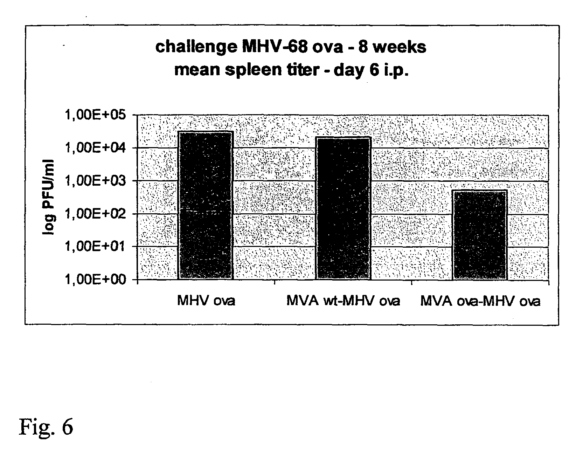

Fig.6 Mean spleen titers of mice challenged with MHV-68-OVA 8 weeks after prime-boost immunization with MVA
   C57B1/6 mice (n=3) were immunized two times within 14 days with MVA-WT or MVA-OVA. 8 weeks after second immunization mice were challenged intraperitoneally with MHV-68-OVA and spleens were harvested at day 6 after challenge.
   Mice immunized with MVA-OVA showed specific reduction of lung titer of about 1.5 log compared to control groups that received MVA-WT or MHV-68-OVA.

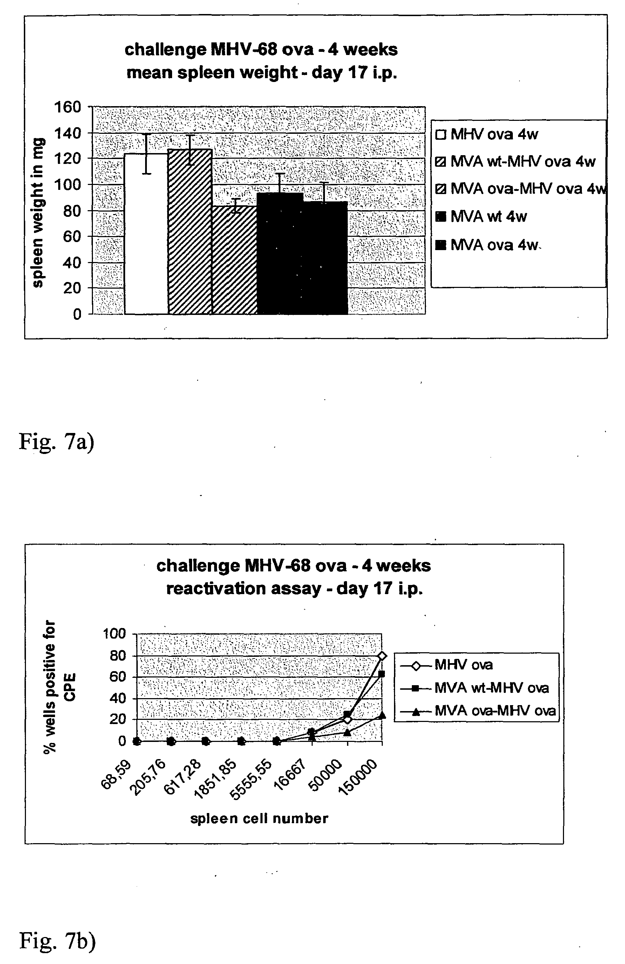

Fig. 7a: Mean spleen weight of mice challenged with MHV-68-OVA 4 weeks after prime-boost immunization with MVA
   C57B1/6 mice (n=3) were immunized two times within 14 days with MVA-WT or MVA-OVA. 4 weeks after second immunization mice were challenged intraperitoneally with MHV-68-OVA and spleens were harvested at day 17 after challenge.
   Mice immunized with MVA-OVA showed specific reduction of spleen weight compared to control groups.
Fig.7b: Reactivation assay of spleen cells after immunization of mice with MVA and challenge with MHV-68-OVA
   Different dilutions of spleen cells of immunized and challenged mice (see Fig.7a) were incubated with NIH3T3 (24 wells per dilution). Wells positive for CPE because of reactivating virus were counted. Corresponding to the result in Fig.7a, less MHV-68-OVA could reactivate from the spleen cells of MVA-OVA vaccinated mice compared to the control group.

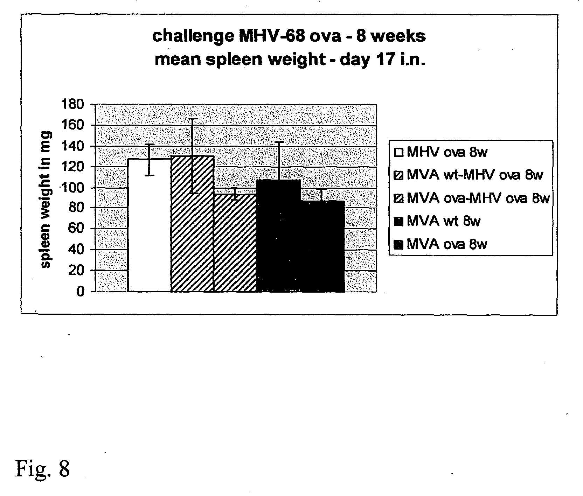

Fig.8 Mean **spleen weight of mice challenged with MHV-68-OVA 4 weeks after prime-boost** immunization with MVA
   C57B1/6 mice (n=3) were immunized two times within 14 days with MVA-WT or MVA-OVA. 8 weeks after second immunization mice were challenged intranasally with MHV-68-OVA and spleens were harvested at day 17 after challenge.
   Mice immunized with MVA-OVA showed specific reduction of spleen weight compared to control groups.

### Examples:

### Material and Methods

### Cell lines

BHK-21 cells were grown in Glasgow-MEM (Biochrom AG, Berlin, Germany) supplemented with 5% FCS, 5% tryptose phosphate broth, 2mM L-glutamine, 100 U/ml Penicillin and 100µg/ml Streptomycin. Ref-Cre cells were maintained in DMEM High Glucose (Cell Concepts, Umkirch, Germany) supplemented with 10% FCS, 2mM L-Glutamine, 100 U/ml Penicillin, 100µg/ml Streptomycin and G418. NIH 3T3 were grown in DMEM High Glucose (Cell Concepts, Umkirch, Germany) supplemented with 10% FCS, 2mM L-Glutamine, 100 U/ml Penicillin and 100µg/ml Streptomycin. MC3T3 (ACC 210, DSMZ, Braunschweig, Germany) were maintained in α-MEM (Biochrom AG, Berlin, Germany) supplemented with 20% FCS, 2mM L-Glutamine, 100 U/ml Penicillin and 100µg/ml Streptomycin.

### Plasmid construction

To construct the recombination plasmid pST76K-SR- MHV-68-ova, the 1.3 kb chicken ovalbumin fragment (excised with EcoRI and XbaI from the plasmid pC-ova) was cloned blunt end into the SmaI/XbaI site of pMCMV4 (kindly provided by Prof. M. Messerle) expression cassette between the MCMV promoter and the polyA signal. The expression cassette (excised by HindIII) was cloned blunt end into the BglII site (position 3846 of the MHV-68 genome) of the plasmid pST76K-SR-MHV-68 . Thus, the ova-expression cassette is flanked on both sides by a homologous sequence of the MHV-68 genome (position 2406-3846 as 5' flank and position 3847-6261as 3' flank). The following two-step mutagenesis procedure was performed as previously described (Adler et al., 2003).

### Reconstitution and characterization of recombinant MHV-68 viruses

To reconstitute the MHV-68 viruses, BHK-21 cells were transfected with 2,5 µg of BAC MHV-68 DNA using Superfect Transfection Reagent (Qiagen). When cells showed total CPE, an aliquot of the supernatant was used to infect REF-Cre cells carrying the Cre recombinase to remove the BAC cassette including the GFP sequence. BAC-cassette free viruses were identified by a limiting dilution assay on BHK-21 cells performed in a 96 well plate. MHV-68-WT and MHV-68-OVA were grown and titered on BHK-21 as previously described (Adler et al, 2000). Briefly, stocks were grown by infecting BHK cells. After showing complete CPE, BHK cells were harvested, resuspended in fresh medium, and the supernatant was used as working stock after two times freezing/thawing the cells and removing cell debris by centrifugation. Virus titers were determined by plaque assay. Briefly, 10 fold dilutions were incubated on BHK-21 cells for 90 min. at 37°C. After removing the inoculum, cells were incubated for 5 days at 37°C with fresh medium containing methylcellulose. Plaques were stained with crystal violet solution.

Genomic DNA of the recombinant MHV-68 was characterized by restriction enzyme analysis, southern blot, low MOI *in vitro* growth curves and *in vivo* growth. To test *in vitro* growth , NIH 3T3 were infected with MOI of 0.1 for 1 hour. After removing the inoculum, cells were incubated with fresh medium at 37°C and 5% CO₂ until supernatant and cells were harvested at different timepoints. Virus titer was determined by plaque assay as described above. Expression of ovalbumin was tested by western blot.

### In vivo experiments

C57BL/6 (B6) were purchased from Charles River Laboratories (Sulzfeld, Germany) and housed in individually ventilated cages (IVC) during the MHV-68 infection period. To characterize MHV-68 viruses *in vivo*, mice were infected intranasally (i.n.) with 5x10⁴ PFU or intraperitoneally (i.p.) with 1x10⁶ PFU. To determine virus titers, lungs were harvested on day 6 after i.n. infection and spleens on day 6 after i.p. infection. For determination of spleen weight and frequency of virus reactivation, spleens were taken on day 17 after i.n. or i.p. infection.

Mice were vaccinated i.p. with 10⁷ PFU MVA-WT or MVA-OVA in 250 µl PBS. Mice were challenged i.n. with 5x10⁴ PFU MHV-68-WT or MHV-68-OVA in 30µl PBS or i.p. with 10⁶ PFU in 250 µl PBS. Prior to i.n. infection mice were anesthetized i.p. with ketamine (50mg/kg) + xylazine (15mg/kg). Timepoints of harvesting lungs and spleens are mentioned above.

To determine virus titers of lungs and spleens, the organs were homogenized in glass douncers. After two times freezing and thawing the homogenates, plaque assay was performed with 10-fold dilutions of the supernatants on BHK-21 cells as described above.

### Limiting dilution reactivation assay

To determine the frequency of cells carrying virus reactivating from latency, threefold dilutions of splenocytes (starting with 1,5x10⁵ cells/well) were plated onto NIH 3T3 (10⁴/well) as described previously.

### Intracellular cytokine staining

MC3T3 were infected for 1 hour with MHV-68-WT or MHV-68-OVA with different MOI and grown o/n. After harvest with 10 mM EDTA in PBS or medium, cells were incubated with splenocytes of vaccinated mice for 2 hours at 37 °C. After adding Brefeldin A (Golgi Plug, PharMingen/Becton Dickinson) at a final concentration of 1µg/ml, cells were incubated for 8 hours at 37°C and then kept on 4°C o/n. Following steps were performed as previously described (Drexler et al., 2003).

Briefly, cells were live/dead stained with ethidium monoazide bromide in PBS/1%FCS and blocked with anti-CD16/CD32 (Fc Block, PharMingen/Becton Dickinson) in PBS/1% FCS/0.02% natriumazide for 20 min. on ice. For cell surface staining cells were incubated with anti-CD8-PE, anti-CD4-PerCP and anti-CD62L-APC for 20 min on ice. Detection of intracellular IFN γ was performed with anti-IFN γ -FITC and anti-IgG-FITC isotype control for 30 min. on ice.

### CTL assay

Splenocytes of naive B6 mice were infected with MHV-68-OVA (MOI of 0.1) for 1.5 h in RPMI (supplemented with 10% FCS, 2mM L-Glutamine, 100 U/ml Penicillin and 100µg/ml Streptomycin and β-mercaptoethanol), washed, irradiated (3000 rad) and incubated with splenocytes of MVA-WT- or MVA-OVA-vaccinated mice for 5 days at 37°C and 5% CO₂. Spleen cells at different E:T ratios were then incubated in round bottomed 96-well plates for 6h with B6 fibroblasts MC3T3 (10⁴/well) that were previously infected with MHV-68-WT or MHV-68-OVA (MOI of 10) for 1.5 h, washed and labelled with ⁵¹Cr (37.5µCi). 30µl of the supernatants were transferred to lumilight plates for determining the ⁵¹Cr release.

### Results

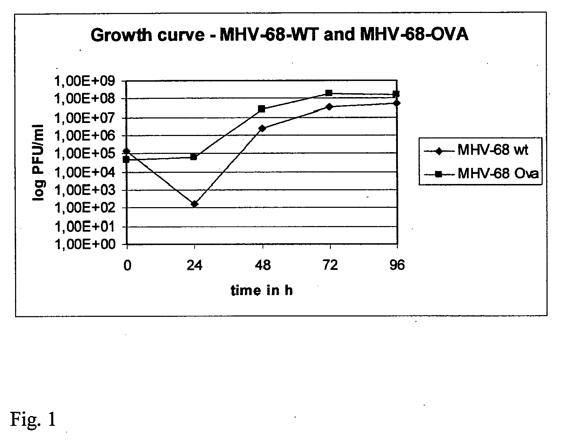

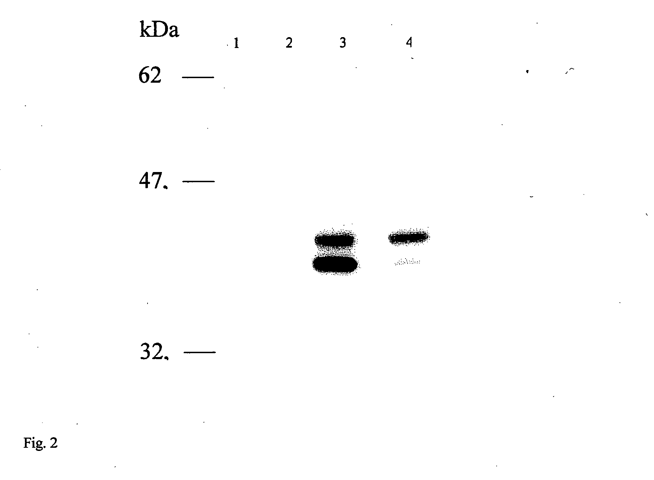

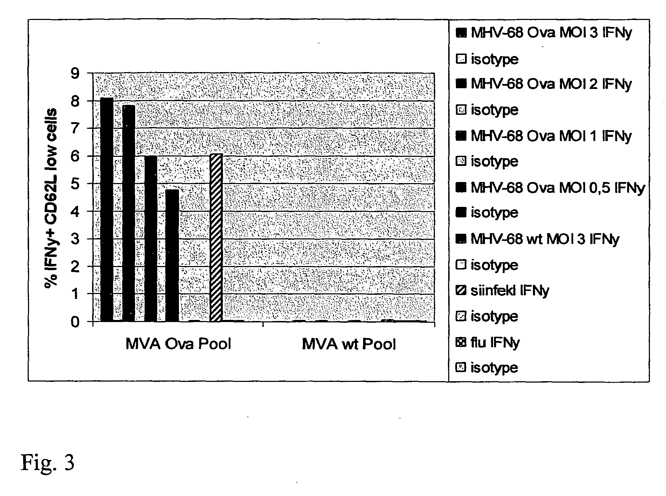

To apply ovalbumin (OVA) as a model antigen we constructed the recombinant virus MHV-68-OVA by BAC-technology and characterized genetic stability and replicative capacity of the virus *in vitro* (Fig. 1) and *in vivo.* We demonstrated the ability of MHV-68-OVA to produce ovalbumin upon tissue culture infections (Fig. 2). Moreover, the use of MHV-68-OVA infected target cells allowed for efficient *ex vivo* amplification of OVA-specific, MHC class I-restricted CD8 T cells derived from MVA-OVA vaccinated C57BL/6 mice (Fig. 3 and 4). Finally, we immunized C57BL/6 mice with MVA-OVA and challenged the animals with MHV-68-OVA testing different time points and routes of infection. Vaccinated mice were infected with MHV-68-OVA but showed reduced viral loads in the acute and latent phase of challenge infection (Fig. 5-8).

These data show the usefulness of the MHV-68 challenge model for the evaluation of recombinant vaccines against persisting virus infections.

### Literature:

Albrecht, J.-C., Nicholas,J., Biller,D., Cameron,K.R., Biesinger,B., Newman,C., Wittmann,S., Craxton,M.A., Coleman,H., Fleckenstein,B., and Honess,R.W. (1992). Primary structure of the Herpesvirus saimiri genome. J. Virol. *66*, 5047-5058.

Chmielewicz, B., Goltz,M., Franz,T., Bauer,C., Brema,S., Ellerbrok,H., Beckmann,S., Rziha,H.J., Lahrmann,K.H., Romero,C., and Ehlers,B. (2003). A novel porcine gammaherpesvirus. Virol. *308*, 317-329.

Cho,Y.-G., Ramer,J., Rivailler,P., Quink,C., Garber,R.L., Beier,D.R., and Wang,F. (2001). An Epstein-Barr-related herpesvirus from marmoset lymphomas. Proc. Natl. Acad. Sci. USA *98*, 1224-1229.

Damania,B. and Desrosiers,R.C. (2001). Simian homologues of human herpesvirus 8. Philos. Trans. R. Soc. Lond B Biol. Sci. *356*, 535-543.

Dittmer,D., Stoddart,C., Renne,R., Linquist-Stepps,V., Moreno,M.E., Bare,C., McCune,J.M., and Ganem,D. (1999). Experimental transmission of Kaposi's sarcoma-associated herpesvirus (KSHV/HHV-8) to SCID-hu Thy/Liv mice. J. Exp. Med. *190*, 1857-1868.

Duboise, M., Guo,J., Czajak,S., Lee,H., Veazey,R., Desrosiers,R.C., and Jung,J.U. (1998a). A role for herpesvirus saimiri orfl4 in transformation and persistent infection. J. Virol. *72*, 6770-6776.

Duboise, S.M., Guo,J., Czajak,S., Desrosiers,R.C., and Jung,J.U. (1998b). STP and Tip are essential for herpesvirus saimiri oncogenicity. J. Virol. *72*, 1308-1313.

Ehlers,B. and Lowden,S. (2004). Novel herpesviruses of Suidae: indicators for a second genogroup of artiodactyl gammaherpesviruses. J. Gen. Virol. *85*, 857-862.

Ehlers, B., Ulrich,S., and Goltz,M. (1999). Detection of two novel porcine herpesviruses with high similarity to gammaherpesviruses. J. Gen. Virol. *80*, 971-978.

Ensser, A., Glykofrydes,D., Niphuis,H., Kuhn,E.M., Rosenwirth,B., Heeney,J.L., Niedobitek,G., Muller-Fleckenstein,I., and Fleckenstein,B. (2001). Independence of herpesvirus-induced T cell lymphoma from viral cyclin D homologue. J. Exp. Med. *193*, 637-642.

Ensser, A., Pfinder,A., Mueller-Fleckenstein,I., and Fleckenstein,B. (1999). The URNA genes of herpesvirus saimiri (strain C488) are dispensable for transformation of human T cells in vitro. J. Virol. *73*, 10551-10555.

Fickenscher, H. and Fleckenstein,B. (2001). Herpesvirus saimiri. Philos. Trans. R. Soc. Lond B Biol. Sci. *356*, 545-567.

Fleckenstein, B. and Desrosiers,R.C. (1982). Herpesvirus saimiri and Herpesvirus ateles. In The Herpesviruses, B.Roizman, ed. (New York and London: Plenum Press), pp. 253-332.

Goltz, M., Ericsson,T., Patience,C., Huang,C.A., Noack,S., Sachs,D.H., and Ehlers,B. (2002). Sequence analysis of the genome of porcine lymphotropic herpesvirus 1 and gene expression during posttransplant lymphoproliferative disease of pigs. Virol. *294*, 383-393.

Grassmann, R. and Fleckenstein,B. (1989). Selectable recombinant herpesvirus saimiri is capable of persisting in a human T-cell line. J. Virol. *63*, 1818-1821.

Huang, C.A., Fuchimoto,Y., Gleit,Z.L., Ericsson,T., Griesemer,A., Scheier-Dolberg,R., Melendy, E., Kitamura,H., Fishman,J.A., Ferry,J.A., Harris,N.L., Patience,C., and Sachs,D.H. (2001). Posttransplantation lymphoproliferative disease in miniature swine after allogeneic hematopoietic cell transplantation: similarity to human PTLD and association with a porcine gammaherpesvirus. Blood *97*, 1467-1473.

Jung, J.U., Choi,J.K., Ensser,A., and Biesinger,B. (1999). Herpesvirus saimiri as a model for gammaherpesvirus oncogenesis. Semin. Cancer Biol. *9,* 231-239.

Knappe,A., Hiller,C., Niphuis,H., Fossiez,F., Thurau,M., Wittmann,S., Kuhn,E.-M., Lebecque,S., Banchereau,J., Rosenwirth,B., Fleckenstein,B., Heeney,J., and Fickenscher,H. (1998a). The interleukin-17 gene of herpesvirus saimiri. J. Virol. *72*, 5797-5801.

Knappe,A., Thurau,M., Niphuis,H., Hiller,C., Wittmann,S., Kuhn,E.-M., Rosenwirth,B., Fleckenstein,B., Heeney,J., and Fickenscher,H. (1998b). T-cell lymphoma caused by Herpesvirus saimiri C488 independently of ie14/vsag, a viral gene with superantigen homology. J. Virol. *72*, 3469-3471.

Lacoste,V., Mauclere,P., Dubreuil,G., Lewis,J., Georges-Courbot,M.-C., and Gessain,A. (2000). KSHV-like herpesviruses in chimps and gorillas. Nature *407,* 151-152.

Moghaddam,A., Rosenzweig,M., Lee-Parritz,D., Annis,B., Johnson,R.P., and Wang,F. (1997). An animal model for acute and persistent Epstein-Barr virus infection. Science *276,* 2030-2033.

Pennington,L.R., Sakamoto,K., Popitz-Bergez,F.A., Pescovitz,M.D., McDonough,M.A., MacVittie,T.J., Gress,R.E., and Sachs,D.H. (1988). Bone marrow transplantation in miniature swine. I. Development of the model. Transplantation *45,* 21-26.

Rickinson,A. (2001). Concluding overview: looking back, looking forward. Philos. Trans. R. Soc. Lond B Biol. Sci. *356*, 595-604.

Rivailler,P., Cho,Y.G., and Wang,F. (2002a). Complete genomic sequence of an Epstein-Barr virus-related herpesvirus naturally infecting a new world primate: a defining point in the evolution of oncogenic lymphocryptoviruses. J. Virol. *76*, 12055-12068.

Rivailler,P., Jiang,H., Cho,Y.G., Quink,C., and Wang,F. (2002b). Complete nucleotide sequence of the rhesus lymphocryptovirus: genetic validation for an Epstein-Barr virus animal model. J. Virol. *76*, 421-426.

Rowe,M., Young,L.S., Crocker,J., Stokes,H., Henderson,S., and Rickinson,A.B. (1991). Epstein-Barr virus (EBV)-associated lymphoproliferative disease in the SCID mouse model: implications for the pathogenesis of EBV-positive lymphomas in man. J. Exp. Med. *173*, 147-158.

Searles,R.P., Bergquam,E.P., Axthelm,M.K., and Wong,S.W. (1999). Sequence and genomic analysis of a Rhesus macaque rhadinovirus with similarity to Kaposi's sarcoma-associated herpesvirus/human herpesvirus 8. J. Virol. *73*, 3040-3053.

Swanton,C., Mann,D.J., Fleckenstein,B., Neipel,F., Peters,G., and Jones,N. (1997). Herpes viral cyclin/Cdk6 complexes evade inhibition by CDK inhibitor proteins. Nature *390*, 184-187.

Ulrich,S., Goltz,M., and Ehlers,B. (1999). Characterization of the DNA polymerase loci of the novel porcine lymphotropic herpesviruses 1 and 2 in domestic and feral pigs. J. Gen. Virol. *80*, 3199-3205.

Wang,F., Rivailler,P., Rao,P., and Cho,Y. (2001). Simian homologues of Epstein-Barr virus. Philos. Trans. R. Soc. Lond B Biol. Sci. *356*, 489-497.

## Claims

1. A screening method for testing the protective capacity of a candidate antigen comprising the steps of:
a) administering a candidate antigen to a non-human mammal;
b) administering recombinant gamma-herpesvirus containing the same antigen to said non-human mammal; and
c) determining the viral load of said recombinant gamma-herpesvirus in said non-human animal and thus the efficacy of the candidate antigen to elucidate an immune response and thereby an immune protection in said non-human mammal.

2. The screening method of claim 1, wherein the non-human animal is selected from the group consisting of primates, pigs, rodents, e.g. mice, dogs, ruminants, e.g. sheeps, and horses.

3. The screening method of claim 1 or 2, wherein the gamma-herpesvirus is selected from the group consisting of Herpesvirus saimiri (HVS), Herpesvirus ateles (HVA), EBV and EBV homologous viruses, for example Lymphocryptovirus, HHV-8 and HHV-8 homologous viruses, porcine gamma-herpesvirus, ovine herpesvirus, for example ovine herpesvirus-2 (OvHV-2) and murine MHV-68.

4. The screening method of one or more of claims 1-3, wherein the candidate antigen is a nucleic acid, from which an antigen is expressed, recombinant bacteria expressing an antigen, antigen in protein form and/or antigen carried by a viral vector.

5. The screening method of one or more of claims 1-4, wherein the viral vector is modified vaccinia virus Ankara (MVA).

6. The screening method of one or more of the preceding claims, wherein the antigen is a pathogenic agent.

7. The screening method of claim 6, wherein the pathogenic agent is a polypeptide or protein derived from the group consisting of viruses, bacteria, protozoa and parasites as well as tumor cells or tumor cell associated antigens and functional parts thereof.

8. The screening method of claim 7, wherein the viruses are selected from the group consisting of influenza viruses, measles and respiratory syncytial viruses, dengue viruses, human immunodeficiency viruses, human hepatitis viruses, herpes viruses, or papilloma viruses.

9. The screening method of claim 7, wherein the protozoa is Plasmodium falciparum.

10. The screening method of claim 7, wherein the bacteria is tuberculosis-causing Mycobacteria.

11. The screening method of claim 7, wherein the tumor cell associated antigen is selected from the group consisting of melanoma-associated differentiation antigens, e.g. tyrosinase, tyrosinase-related proteins 1 and 2, of cancer testes antigens, e.g. MAGE-1,-2,-3, and BAGE, and of non-mutated shared antigens overexpressed on tumors, e.g. Her-2/neu, MUC-1, and p53.

12. The screening method of one or more of the preceding claims, wherein the candidate antigen and the gamma-herpesvirus are administered to the non-human mammal by intravenous, intranasal, intraperitoneal, buccal, subcutaneous, intramuscular, peroral administration or by inhalation.

13. The screening method of one or more of the preceding claims, wherein the acute protective capacity of the candidate antigen is tested within a time period of between 4-12, preferably 5-10, most preferably about 6 days following step b).

14. The screening method of one or more of the preceding claims, wherein the chronic protective capacity of the candidate antigen is tested within a time period of between 10-150, preferably 15-20, most preferably about 17 days following step b).

15. The screening method of claim 13, wherein the acute protective capacity is tested by determining the titer of gamma-herpesvirus in lung and/or spleen tissue/cells or in other organs, which contain gamma-herpesvirus infected cells.

16. The screening method of claim 14, wherein the chronic protective capacity is tested by determining the degree of spleen enlargement and/or by determining the viral load in splenocytes and/or in dendritic cells and/or macrophages as measured by *ex vivo* reactivation and/or by PCR.

17. The screening method of one or more of the preceeding claims, wherein the protective capacity is determined ex vivo by:
- providing splenocytes from a non-human mammal which was infected with a candidate antigen;
- contacting said splenocytes with cells infected with recombinant gamma-herpesvirus containing the same antigen; and
- determining the degree of cytotoxic T cell activation and/or helper T cell function and/or antibody production.

18. The screening method of one or more of the preceeding claims, wherein the candidate antigen in step a) is contained in a vaccine.

19. An antigen or a vaccine having protective capacity against a disease identified by the screening method of one or more of claims 1-18.
